# EUROPEAN PATENT APPLICATION

(11) **EP 2 251 662 A1**
(43) Date of publication of application: **17.11.2010**
(21) Application number: 09006101.1
(22) Date of filing: 05.05.2009
(51) Int. Cl.: G01L 5/16, B06B 1/06

(54) **Combined force and ultrasound sensor and associated method**

(71) Applicant: ETH Zurich, 8092 Zürich (CH)
(72) Inventor: Szekely, Gabor, 8032 Zürich (CH); Kosa, Gabor, 6963 Pregassona (CH)

(57) **Abstract**

A sensor device (40) for force measurements and ultrasonic imaging is disclosed. The device comprises one or more piezoelectric elements (11, 12) consisting of a piezoelectric material having a first acoustic impedance, and a set of electrodes (43, 44), the electrodes arranged on the piezoelectric elements. The electrodes are arranged in a manner to allow to excite and detect an ultrasonic sound field at least along the axial direction of the device, and to allow to collect and distinguish charges on the piezoelectric elements resulting from forces along at least the lateral directions perpendicular to the axial direction. An acoustic impedance matching element (18) may be arranged on an ultrasound excitation surface of at least one of said piezoelectric elements for matching an acoustic impedance of the piezoelectric material to an acoustic impedance of a surrounding medium. The sensor device may be connected to a signal processing device to obtain force and ultrasound information.

## Description

### TECHNICAL FIELD

The present invention relates to a sensor device that is capable of carrying out ultrasound measurements on an object disposed in the vicinity of the sensor device and of determining forces acting on the sensor device, and to an associated method.

### PRIOR ART

In certain surgical procedures, in particular in minimally invasive surgery, it is necessary to obtain information about the tissue in the vicinity of a surgical instrument without being able to visually inspect the tissue. Such information may include thickness of the tissue, tissue density, tissue structure or velocity of body fluids. It has been proposed to use ultrasound at least for axial ranging in such circumstances. However, for meaningful interpretation of such measurements, it is often required that the direction of ultrasound excitation relative to the tissue be precisely known, e.g., that the ultrasound transducer be placed into a precisely defined contact with a surface of the tissue in a known orientation relative to the surface. There is thus a need for a sensor device that is not only able to provide information from ultrasound measurements, but also information about the magnitude and/or the direction of a contact force between the device and an anatomical surface.

US 6,695,808 discloses several embodiments of sensors employing one or more force transducers for determining the magnitude and, in some embodiments, the direction of contact forces between the sensor and an anatomical surface. A change in the contact force results in a change of either Ohmic resistance, capacitance or inductivity of the force transducer. The document further proposes to combine such force transducers with an ultrasonic transducer, such as the transducer disclosed in US 6,024,703, to perform axial ranging. However, these known devices are relatively complicated and require very precise fabrication of a relatively large number of different parts.

WO 2009/007047, whose contents are incorporated herein in their entirety by reference, discloses a piezoelectric device for determining loads (forces and/or torques) associated with multiple degrees of freedom. The sensing principle is piezoelectric. Particular loads generate associated surface charge distributions on a piezoelectric bimorph, which are collected by electrodes arranged in a manner to distinguish between charge distributions corresponding to multiple degrees of freedom. Only applications for determining slowly varying loads on timescales much slower than the ultrasound time scale are disclosed.

European patent application 08 022 373.8, filed on 23 December 2008, was filed before the priority date of the present patent application, but will be published only thereafter. Its contents are herein incorporated in their entirety by reference. This document discloses a hollow piezoelectric force sensor for determining forces and/or torques corresponding to multiple degrees of freedom. Again, only a determination of essentially static loads is disclosed.

### SUMMARY OF THE INVENTION

It is an object of the present invention to provide a device that is capable of performing ultrasound measurements along at least an axial direction as well as of determining forces at least along the lateral directions perpendicular to the axial direction, which may be manufactured easily and at low cost.

This and other objects are solved by a sensor device according to claim 1. Advantageous embodiments are laid down in the dependent claims.

Thus, a sensor device is provided. The sensor device comprises one or more piezoelectric elements consisting essentially of a piezoelectric material having a first acoustic impedance. A set of electrodes is arranged on the piezoelectric elements. These electrodes are arranged in a manner to allow to excite an ultrasound field at least along a first direction. In addition, the electrodes are arranged so as to allow to collect and distinguish charges on the piezoelectric elements resulting from forces along at least a second and a third direction perpendicular to the first direction. In advantageous embodiments, an acoustic impedance matching element is applied to an ultrasound excitation surface of at least one of the piezoelectric elements for matching said first acoustic impedance to a second acoustic impedance of a surrounding medium.

The present invention proposes to combine, in single unit, a piezoelectric ultrasound transducer with a biaxial piezoelectric force sensor for detecting lateral forces. The working principle of such a sensor device is entirely by the piezoelectric effect. Such devices may be mass-produced at low cost and do not require assembly of a large number of mechanical components. The device is suitable to be used in medical applications, in particular in surgical procedures requiring the positioning of a medical instrument relative to an anatomical surface, but its use is in no way restricted to such medical applications, and the device may also be used in other applications such as material testing.

The lateral forces to be detected in the second and third directions have different properties than the ultrasonic vibrations in a number of aspects. In a first aspect, these forces may be generally non-oscillatory, whereas ultrasonic vibrations are oscillatory in nature. In another aspect, the time scales involved are very much different. Whereas ultrasonic vibrations are in the ultrasound frequency range (above approximately 30 kHz, preferably above approximately 200 kHz, typically 1 MHz - 50 MHz), the lateral forces will generally be quasi-static in nature on these time scales, with substantial variations at most on a time scale that is at least one order of magnitude longer, typically above a few milliseconds.

In order to improve the sensitivity of the device for ultrasound applications, the device advantageously comprises an acoustic impedance matching element. The acoustic impedance Z is defined as the product of density ρ and sound velocity c:

Z = ρc.

Acoustic impedance matching elements are *per se* well known in the art of ultrasonic transducers. They typically comprise a layer of an acoustically "softer" material (a material having a lower acoustic impedance, in particular a lower density and/or a lower sound velocity) than the material from which the bulk of the transducer is made. The acoustic impedance matching element may thus comprise at least one layer of a material having a third acoustic impedance lower than the first acoustic impedance. The layer may advantageously have a thickness of approximately one quarter of a wavelength of the ultrasound field in the layer or of an odd multiple thereof. While the presence of an acoustic impedance matching element is clearly advantageous, the invention is not restricted to the presence of such an element, and also encompasses embodiments that do not have such an element.

In some embodiments, the sensor device may have a generally elongated shape, having a long axis, the length along the long axis being larger than the dimensions perpendicular to the long axis, and in this case the first direction will be the long axis of the sensor device. The acoustic impedance matching element will then be advantageously arranged at a distal end of the device with respect to the long axis.

In some embodiments, the sensor device may be combined with a signal processing device electrically coupled to the electrodes to obtain a complete sensor system. The signal processing device may then comprise:
an ultrasound module adapted to provide voltages to said electrodes in a manner to excite an ultrasonic vibration of at least one of said piezoelectric elements, to detect charge oscillations on the same piezoelectric element corresponding to at least one ultrasonic return signal in response to said ultrasonic vibration, and to determine an output signal representing said ultrasound return signal; and
a load evaluation module adapted to detect charges collected by the electrodes on at least one of said piezoelectric elements and to determine, from said charges, at least two output signals representing said forces along the second and the third direction.

The signal processing device may be integrated in a single unit with the sensor device or may be remote from the sensor device. It may be constructed entirely in analog hardware or may comprise both analog and digital components. In particular, the signal processing device may comprise a processor programmed in software or firmware to perform part of the signal processing involved.

Due to the different types of signals and the different time scales involved, the ultrasound module and the load evaluation module will typically involve different electronic setups. Suitable ultrasound modules are well known in the art from ultrasound ranging and imaging. In particular, the ultrasound module may comprise an oscillator, a receiver and an active or passive switch operable to alternatively connect the oscillator (possibly via an amplifier and a pulse shaper) or the receiver to the electrodes. The receiver may, in some embodiments, be connected to a display that provides a visual indication of the output signal, e.g., by simply displaying signal amplitude versus echo time in A mode, or it may be connected to a digital processor for further signal processing. In particular, from the output signal a measure for a thickness of an object along at least one direction, a tissue density along at least one direction or a distance between said piezoelectric device and an object along at least one direction may be determined. It is also possible to provide a facility for Doppler ultrasound measurements, e.g. to determine blood flow velocities, as it is generally known in the art. For rejecting signals outside the ultrasound range of interest, the ultrasound module may comprise a bandpass filter centered on a predetermined operating frequency in the ultrasound frequency range.

Also suitable load evaluation modules are known. In some embodiments, such a module may comprise a commercially available data-acquisition card with a sufficiently high input impedance, plugged into a general-purpose computer, to whose inputs the electrodes are connected directly or via an analog signal conditioning circuit. For rejecting ultrasound signals and high-frequency noise, the load evaluation module may comprise a lowpass filter having a cutoff frequency below the center frequency of the bandpass in the ultrasound module and preferably below approximately 20 kHz.

For ultrasound excitation and detection, the sensor device is preferably adapted to be operated at or near an acoustic resonance. In particular, it is preferred that the piezoelectric element has at least one acoustic (i.e., longitudinal vibrational) resonance at a resonance frequency in the ultrasound range. Then, on the one hand, it is preferred that the ultrasound module is specifically adapted to excite an ultrasonic vibration with an operating frequency at or near the resonance. On the other hand, it is preferred that the thickness of the acoustic impedance matching layer is approximately one quarter of the wavelength of an ultrasound field in the matching element at the resonance frequency or an odd multiple thereof. Matching the operating frequency and/or the thickness of the matching layer to the resonance frequency provides for an improved efficiency and signal-to-noise (S/N) ratio.

In preferred embodiments, each piezoelectric element is employed for both ultrasound excitation/detection and force detection, instead of providing an independent force sensor and an independent ultrasound transducer. This yields a particularly compact sensor device that may be manufactured easily. In particular, it is possible to employ only a single piezoelectric element (such as a tube, a bimorph or a multimorph) for these functions. In more general terms, the set of electrodes may comprise a first subset of electrodes for exciting and detecting ultrasonic vibrations (which may then be connected to the ultrasound module of the processing device) and a second subset of electrodes for collecting charges resulting from forces (which may then be connected to the load evaluation module), and at least some of the electrodes from the first and second subset may be disposed on at least one common piezoelectric element carrying electrodes of both subsets, integrating the functions of force detection and ultrasound on the same piezoelectric element(s).

In some embodiments, the first and second subsets of electrodes may overlap, i.e., the first and second subsets may have some or even all electrodes in common. In other words, some or all electrodes may be employed for both force determination and ultrasound. In other embodiments, however, these functions may be assigned to entirely separate electrodes, which allows to optimize the electrodes in each subset for their specific respective functions.

To simplify fabrication of the sensor, it is advantageous if each electrode for force determination and each electrode for ultrasound has a normal vector that is substantially perpendicular to the first direction, i.e. if all electrodes are applied on the lateral sides of the device. This implies that, for ultrasound excitation and detection, the device is operated in transverse mode.

If, on the other hand, entirely separate piezoelectric elements are used for force determination and for ultrasound excitation/detection, the piezoelectric element(s) for ultrasound excitation/detection may be positioned and oriented in such a manner that these piezoelectric element(s) may be operated in thickness mode for ultrasound excitation, which yield an improved efficiency over embodiments wherein the piezoelectric elements operate in transverse mode. In other words, the sensor device may comprise one or more first piezoelectric elements for force determination, on which the first subset of electrodes is disposed, and one or more second piezoelectric elements for ultrasound excitation and detection, on which the second subset of electrodes are disposed. The piezoelectric elements have a predetermined electric polarization direction. Then the piezoelectric elements are preferably disposed such that the polarization direction of the first piezoelectric element(s) is substantially perpendicular to the polarization direction of the second piezoelectric element(s).

The electrodes for the ultrasound functionality may be arranged to permit not only excitation/detection along a single axial direction, but also to allow to adjust a direction of said sound field relative to said piezoelectric element. In particular, at least one group of electrodes may be arranged as a one-dimensional array, i.e., arranged in a repetitive arrangement, along a direction that is substantially orthogonal to the first direction. In this case, the ultrasound module is adapted to adjust the direction of said sound field relative to said piezoelectric element, in particular, by providing differently phased signals to the electrodes in the array, as it is well known in the art of ultrasound imaging ("one-dimensional phased array"). In particular, such an array may be used for sound beam shaping and/or focusing. Of course, it is also possible to arrange and operate the electrodes in a two-dimensional phased array, as it is also known in the art.

In advantageous embodiments, the sensor device is also capable to determine axial forces in addition to lateral forces. In other words, it is preferred that the electrodes are arranged on said piezoelectric elements in a manner to allow to collect and distinguish charges on said piezoelectric elements resulting from forces along the first direction, in addition to forces along the second and third directions. The load evaluation unit will then be specifically adapted to determine, from said charges, at least three output values representing said forces along the first, second and the third direction.

The sensor device may, in some embodiments, be capable to determine torque around at least one of the lateral directions, in addition to forces. In other words, the electrodes may be arranged in a manner that allow to collect and distinguish charges on the piezoelectric elements resulting from torque around at least one of the second and a third directions, in addition to forces along the second and third directions. The load evaluation unit will then be specifically adapted to determine, from said charges, at least an additional output value representing said torque.

In advantageous embodiments, piezoelectric elements as described in the document WO 2009/007047 or in European patent application 08 022 373.8 mentioned above may be used for load determination, and the electrodes for determining loads may be arranged as disclosed therein. Explicit reference is made to these documents for suitable piezoelectric elements and electrode arrangements and for considerations concerning the derivation of output values representing forces and/or torques from the electrode charges and/or voltages. Additional electrodes may then be provided for ultrasound excitation and detection.

In particular, the sensor device may comprise a piezoelectric bimorph having a first and a second piezoelectric layer,
wherein the set of electrodes comprises a central electrode being arranged between the first and the second piezoelectric layer, and
wherein the set of electrodes comprises a plurality of electrodes arranged on outer surfaces of said first and second layer facing away from said central electrode.

The piezoelectric bimorph may have a long axis substantially along said first direction and may have a proximal and a distal end relative to the long axis. In particular, the bimorph may have an elongated shape. The acoustic impedance matching element will then be advantageously arranged on a distal end of the bimorph. The electrodes arranged on the outer surfaces will then comprise electrodes for exciting an ultrasound field and for collecting charges resulting from forces, and the respective subsets of electrodes may overlap, as described above. The bimorph will be operated in transverse mode for ultrasound excitation and detection, and the ultrasound module may be specifically adapted for exciting transverse mode vibrations substantially along the long axis and for detecting such transverse mode vibrations resulting from an ultrasound return signal.

In one particular embodiment, the set of electrodes comprises:
a first group of at least two electrodes for determining loads arranged on the outer surface of the first piezoelectric layer;
a second group of at least two electrodes for determining loads arranged on the outer surface of the second piezoelectric layer, each electrode from the second group facing an electrode of substantially identical size and shape of the first group; and
a third group of at least two electrodes for exciting the ultrasound field, the third group being arranged on said first and second piezoelectric layers in a distal end region of said bimorph, distally from the first and second groups.

In all bimorph embodiments, the central electrode may be hollow, providing a through-hole along the long axis, as disclosed in European patent application 08 022 373.8, and explicit reference is made to this document for disclosing a bimorph having a hollow central electrode.

In other embodiments, the piezoelectric elements may comprise a hollow cylindrical tube made from a piezoelectric material, the tube having a long axis, an inside peripheral surface and an outside peripheral surface. The set of electrodes may then comprise at least one inner electrode arranged on said inside peripheral surface and a plurality of electrodes arranged on said outside peripheral surface, or vice versa.

In one particular embodiment, the set of electrodes comprises:
a first group of four electrodes for determining loads, distributed over a circumference of a peripheral surface of the tube; and
a second group of at least one electrode for exciting the ultrasound field, the second group being arranged on the outer surface of the tube in a distal end region of said tube, distally from said first group.

The present invention also relates to a method of use of a single piezoelectric sensor device for ultrasound measurements as well as force measurements. In general terms, the invention provides a method for determining information about an object along a first direction and force information along at least two directions perpendicular to the first direction, the method comprising:
providing a sensor device comprising one or more piezoelectric elements and a set of electrodes, the electrodes being arranged on said piezoelectric elements in a manner to allow to excite and detect an ultrasonic sound field at least along a first direction and to allow to collect and distinguish charges on said piezoelectric elements resulting from forces along at least a second and a third direction perpendicular to the first direction;
providing voltages to said electrodes in a manner to excite an ultrasonic vibration of said piezoelectric element;
detecting charge oscillations on said piezoelectric elements corresponding to at least one ultrasonic return signal in response to said ultrasonic vibration; and
detecting charges collected by the electrodes and determining, from said charges, at least two output values representing said forces along the second and the third direction.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a sensor device according to a first embodiment of the present invention in a schematic perspective view;
- Fig. 2: shows a sensor device according to a first embodiment of the present invention in a schematic perspective view;
- Fig. 3: shows a schematic diagram of the sensor device of Fig. 1, together with a control device;
- Fig. 4: shows a sensor device according to a third embodiment of the present invention in a schematic perspective view;
- Fig. 5: shows a sensor device according to a fourth embodiment of the present invention in a schematic perspective view;
- Fig. 6: shows a schematic diagram of the sensor device of Fig. 4, together with a control device;
- Fig. 7: shows a sensor device according to a fifth embodiment of the present invention in a schematic perspective view;
- Fig. 8: shows a sensor device according to a sixth embodiment of the present invention in a schematic perspective view;
- Fig. 9: shows a schematic diagram of the sensor device of Fig. 7, together with a control device;
- Fig. 10: shows a sensor device according to a seventh embodiment of the present invention in a schematic perspective view;
- Fig. 11: shows a sensor device according to a eighth embodiment of the present invention in a schematic perspective view;
- Fig. 12: shows a sensor device according to a ninth embodiment of the present invention in a schematic sectional view;
- Fig. 13: shows the sensor device of Fig. 12 in a schematic back view;
- Fig. 14: shows a schematic diagram illustrating a possible embodiment of an ultrasound module; and
- Fig. 15: shows a schematic diagram illustrating a possible embodiment of a receiver.

### DESCRIPTION OF PREFERRED EMBODIMENTS

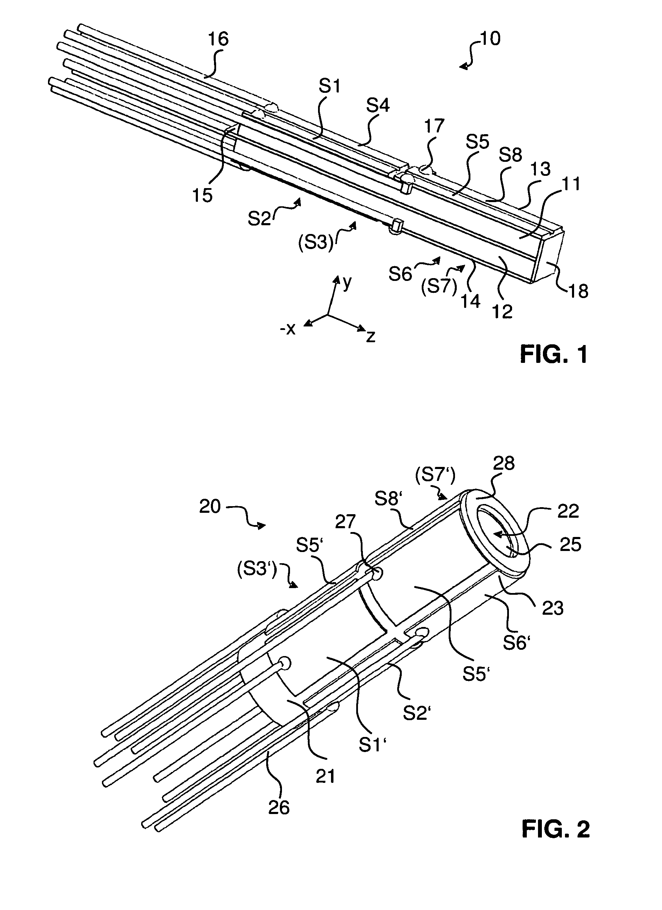

Figure 1 illustrates a first embodiment of a sensor device. The sensor device 10 comprises an elongated piezoelectric bimorph defining a long axis, the bimorph comprising a first (upper) piezoelectric layer 11 and a second (lower) piezoelectric layer 12. The piezoelectric layers are generally flat and plate-like. These layers may consist of any material exhibiting a sufficiently strong piezoelectric effect, in particular a ceramic material such as PZT (lead zirconium titanate) or a polymer material such as PVDF (polyvinylidene fluoride). PZT plates are preferred. The electric polarization of each layer has a direction perpendicular to the layer plane, and the polarizations of the two layers may be antiparallel or parallel. A central electrode 15 is disposed between the layers, four upper electrodes 13 are disposed on the outer surface of the upper layer, and four lower electrodes 14 are disposed on the outer surface of the lower layer, the outer surfaces being defined as those surfaces that face away from the central electrode. All electrodes are electrically conductive and in contact with the piezoelectric layer(s) on which they are disposed. A connecting wire 16 is bonded to each electrode at a solder point 17. The electrodes on both layers are of identical rectangular shape and of identical size. They are arranged in identical quadrilateral arrangements. The electrodes are designated as S1-S8. The first four electrodes, S1-S4, are closer to the proximal end of the device, whereas electrodes S5-S8 are closer to the distal end of the device.

Directions are identified as follows: The long axis is designated as the z direction. The y direction is the direction normal to the electrodes. The direction perpendicular to the z direction and parallel to the electrodes is designated as the x direction.

The sensor's working principle is piezoelectric. Force or torque applied on the piezoelectric bimorph creates axial and/or bending stresses (depending on the direction of the external load). Strain is created in the beam and converted by the direct piezoelectric effect into electric displacement. Change in electric displacement accumulates charge on the sensors electrodes which may be measured by a signal processing unit.

The general setup of this device is very similar to the setup of the force/moment sensors disclosed in WO 2009/007047, and reference is made to that document for further details and for alternative electrode arrangements. Reference is further made to that document for disclosing how such a device may be used to determine and distinguish loads associated with different degrees of freedom (DOF), in particular, with forces along different directions and torques around different directions.

In particular, if only forces along the x, y and z direction need to be detected and distinguished, but need not be distinguished from torques, it is sufficient if only the four distal electrodes S5-S8 are present, and proximal electrodes S1-S4 may be left away.

In addition to determining forces and/or torques, the device may also be used to excite and detect an ultrasound field. To this end, AC voltages having a frequency in the ultrasound frequency range may be applied to some of all of the electrodes with appropriate phase. In particular, if the bimorph layers have antiparallel polarizations, an AC voltage may be applied to all electrodes S1-S8 or to the distal electrodes S5-S8, relative to the central electrode, with identical phase. Such an AC voltage gives rise to oscillatory electric fields in the piezoelectric layers along the y direction, which are converted into dimensional changes by the piezoelectric effect. In particular, the voltage will lead to contractions and elongations along the z direction through the piezoelectric coefficient d₃₁ (transverse mode). In this manner, a longitudinal acoustic vibration of the device at ultrasound frequency is obtained. By the inverse effect, such vibrations may also be detected.

For ultrasound measurements, the device may be operated as follows: first, an ultrasonic vibration is generated during a short excitation pulse having a duration of a few milliseconds. The pulse is then stopped, and returned ultrasound signals are now detected by measuring the AC voltage on the electrodes at the excitation frequency. The pulse will generate sound waves that start to propagate into the surrounding medium. The waves will be reflected at interfaces, where a change in acoustic impedance occurs, and the returned sound signals are recorded. In particular, the time lag between the excitation pulse and any returned sound signal is proportional to the distance travelled by this signal and thus provides information about the axial distance of the interface at which the pulse was reflected. The magnitude and the frequency of the return signal provides additional information. This principle and many refinements of such methods are well known in the art of ultrasound diagnostics.

In order to improve the efficiency of the device, an acoustic impedance matching element in the form of a matching layer 18 is applied to the distal end face (the "ultrasound excitation surface") of the bimorph. The matching layer is made of a softer material than the piezoelectric material, e.g., of epoxy. It preferably has a thickness corresponding to a quarter wavelength of the sound waves within the matching layer.

Figure 2 illustrates a second embodiment of a sensor device. The device 20 comprises a tube 21 of circular-cylindrical shape, made of a piezoelectric material like PZT. An inner electrode 25 (reference electrode) is applied to the inner peripheral surface of the piezoelectric tube 21. A central bore 22 extends through the device, the bore being bounded by the inner electrode 25. Eight outer electrodes 23 are distributed over the outer peripheral surface of the tube 21. These electrodes will in the following be designated as electrodes S1' to S8'. A connecting wire 26 is connected to each electrode at a solder point 27.

For this device, directions are identified as follows: The longitudinal (cylinder) axis of the device is designated as the z axis. The mutually orthogonal transverse directions perpendicular to the longitudinal axis are designated as the x and y directions, respectively. Like the device of the first embodiment, also the device shown in Fig. 2 is suited for distinguishing and determining forces applied to it along the longitudinal (z) axis or along any of the two transverse (x, y) axes, and for distinguishing and determining torques (moments) applied to it around the transverse (x, y) axes. In other words, the device is adapted to be used as a sensor for distinguishing and determining loads in the form of forces and torques associated with five different degrees of freedom. For further details and for alternative electrode arrangements, reference is made to European patent application 08 022 373.8 mentioned above. In particular, if only forces along the x, y and z directions need to be determined and distinguished, it is sufficient if only the four distal electrodes S5-S8 are present.

Like in the first embodiment, the device may also be operated as an ultrasound sensor in transverse mode, by applying AC voltages to the electrodes with some predetermined phase relationship. In particular, AC voltages may be applied relative to the inner electrode to all electrodes or only to the distal electrodes S5'-S8', the voltages having identical phase. After the pulse, such voltages may be measured to determine an ultrasound return signal.

Again, an acoustic impedance matching element in the form of a matching layer 28 is applied to a distal end face of the device, serving as the ultrasound excitation surface. This surface and the matching layer thereon have an annular shape. This provides for focusing of the ultrasound field emitted from the surface by the Huygens principle. The focus will be located along the axial direction at a certain axial distance from the surface, the distance depending on the dimensions of the device and on the operating frequency.

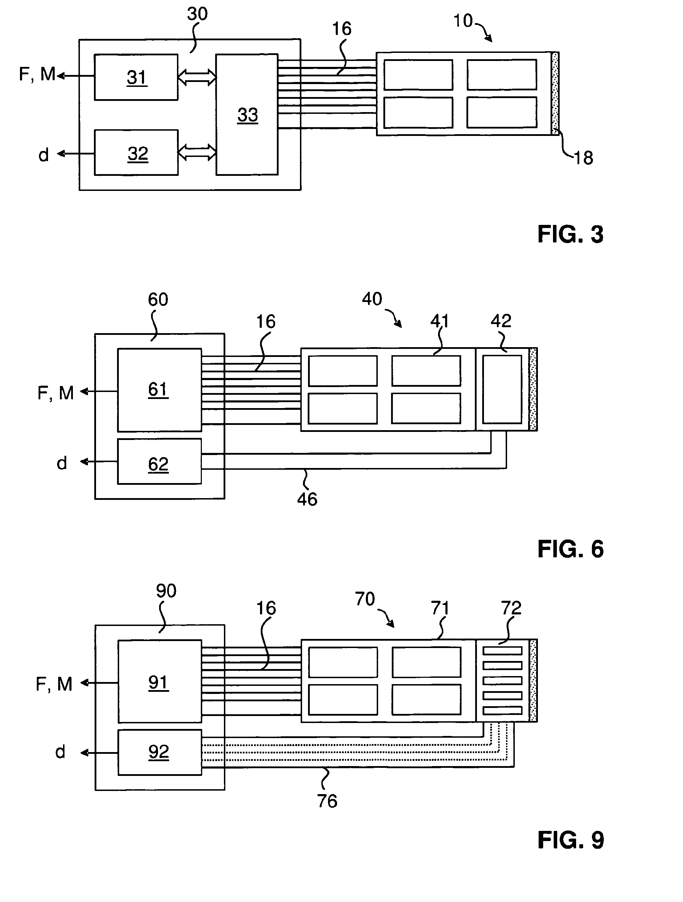

Figure 3 illustrates a possible operational scheme of devices of the type of the first two embodiments. A signal processing device 30 comprises a load evaluation module 31 and an ultrasound module 32. The load evaluation module 31 serves to detect charges on the sensor device according to the first or second embodiment by measuring voltages between the electrodes, and to derive output values corresponding to forces F and possibly moments M. It rejects ultrasound signals by passing the signals through a lowpass filter with a cutoff below 20 kHz. The ultrasound module 32 serves to excite and detect ultrasonic vibrations, as described above, and to determine an output value which might, e.g., represent depth or thickness d, as it is well known in the art. Both modules are connected to a multiplexer module 33, which alternatively connects the load evaluation module 31 and the ultrasound module 32 to the sensor device 10, according to some switching scheme (multitasking scheme), which may be provided by a control unit integrated with the multiplexer module or by an external controller.

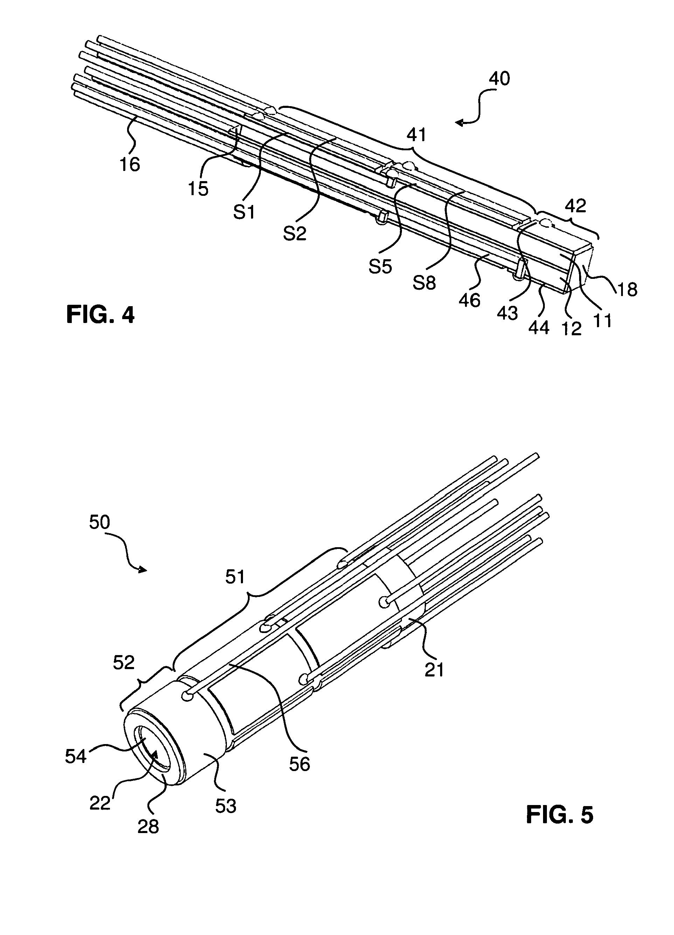

In the first two embodiments, the same electrodes are used for load determination and for ultrasound. In contrast, Figure 4 illustrates an embodiment of a sensor device 40 wherein there are separate subsets of electrodes for these functionalities. A first subset comprises two electrodes 43 and 44 in a distal ultrasound section 42 of the device, these electrodes being connected to wires 46 and serving only for ultrasound generation and detection. A second subset of electrodes comprises electrodes S1-S8 as in the first embodiment and is disposed in a proximal force/moment sensing section 41. The two subsets are disposed on the same single bimorph and share the same piezoelectric layers 11 and 12. This arrangement allows to optimize the electrode configurations of the two subsets to their respective specific tasks.

A fourth embodiment is illustrated in Fig. 5. The sensor 50 employs a single piezoelectric tube 21, as in the second embodiment. In a proximal force/moment sensing section 51, electrodes are arranged on the tube 21 like in the second embodiment. In a distal ultrasound section 52, an outer annular electrode 53 and an inner annular electrode 54 are disposed on the tube 21 for ultrasound excitation and detection. The inner electrode 54 may or may not be separate from the inner electrode 25 on the inside of the tube in the force/moment sensing section.

Operation of such devices is schematically illustrated in Fig. 6. A signal processing device 60 again comprises a load evaluation module 61 and an ultrasound module 62, which are generally set up as in the embodiment of Fig. 3. However, no multiplexer module is needed, and forces/moments may be determined simultaneously and independently of ultrasound operation.

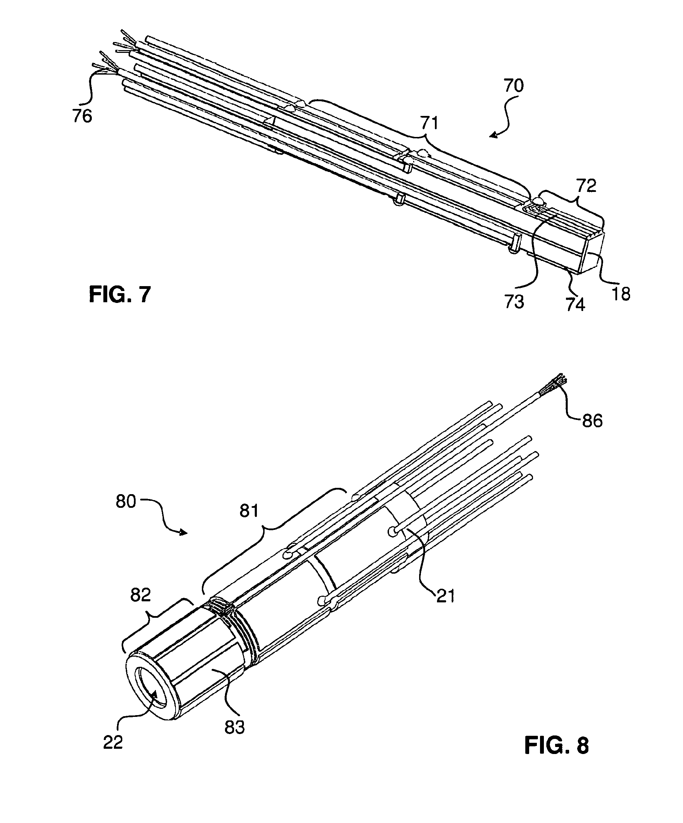

Figure 7 illustrates a fifth embodiment of a sensor 70. Again, there is a proximal force/moment sensing section 71 and an ultrasound section 72. In the ultrasound section, a group of upper electrodes 73 and a group of lower electrodes 74 are disposed on the outer surface of the bimorph. Each electrode is connected to a wire 76, these wires forming a bundle. Each group is arranged in a repetitive arrangement along the x axis, i.e., each group forms a linear array of a plurality of independent electrodes. By providing voltages to these electrodes with a particular phase relationship, the ultrasound beam emitted from the device may be steered into different directions within the x-z plane of the bimorph, and echoes from different directions may be distinguished. These principles as such are well known in the art of ultrasound ranging/imaging.

Similarly, Fig. 8 illustrates a sixth embodiment of a sensor 80. A proximal force/moment sensing section 81 is set up like in the second and fourth embodiments. A distal ultrasound section 82 comprises an array of electrodes 83 arranged in a repetitive arrangement along a circumferential direction around the long axis of the device.

Operation of such devices having a phased array is illustrated in Fig. 9. A signal processing device 90 comprises a load evaluation module 91 as discussed above, and an ultrasound module 92. This module is adapted to provide the electrodes in the ultrasound section with AC voltages having a predetermined phase relationship to steer the excited ultrasound beam, and to take the phase relationship of the return signals into account when detecting the return signals, to derive directional information from the return signals.

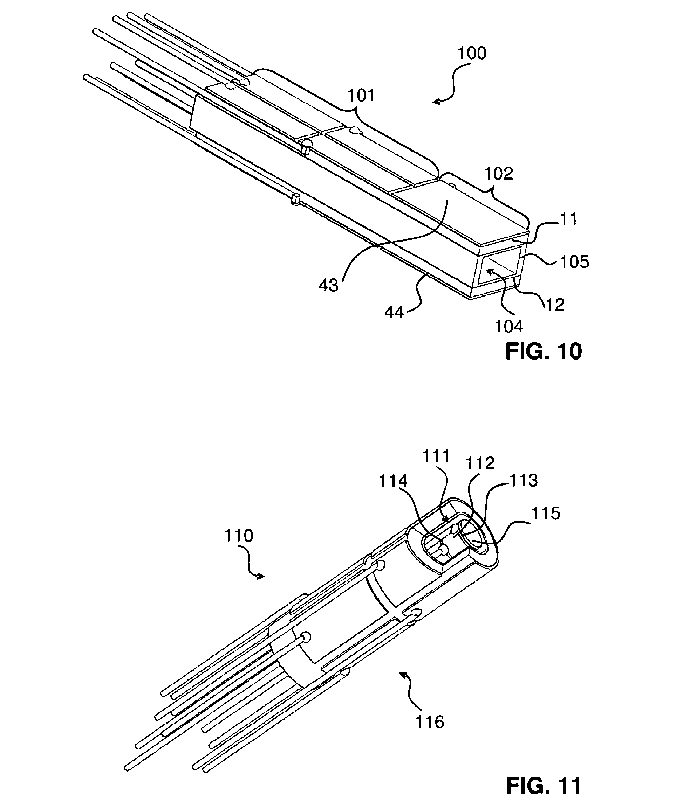

Figure 10 illustrates a seventh embodiment of a sensor 100. Again, the sensor has a force/moment sensing section 101 and an ultrasound section 102, similar to the device of Fig. 4. However, the central electrode 105 takes the form of a hollow cuboid, having a longitudinal through-hole 104. This allows to pass a tool through the through-hole, as in the tubular embodiments of the second, fourth and sixth embodiments.

Figure 11 illustrates an eighth embodiment of a sensor 110, wherein a separate force/moment sensor 116 (of similar configuration as in the second embodiment, except for the absence of an impedance matching layer) and a separate ultrasound transducer 111 are provided. The ultrasound transducer 111 comprises a piezoelectric element 112 in the form of a short cylinder coaxial with the tube sensor 116, whose proximal and distal ends are provided with electrodes 113, 114. An acoustic impedance matching layer 115 is provided on the distal electrode 113. The electric polarization of the piezoelectric element 112 is along the axial (z) direction, whereas the electric polarization of the piezoelectric tube of sensor 116 is in the radial direction, and therefore the ultrasound transducer may be operated in thickness (d₃₃) mode, yielding a generally improved efficiency. The ultrasound transducer is provided inside the bore of the tube sensor 116.

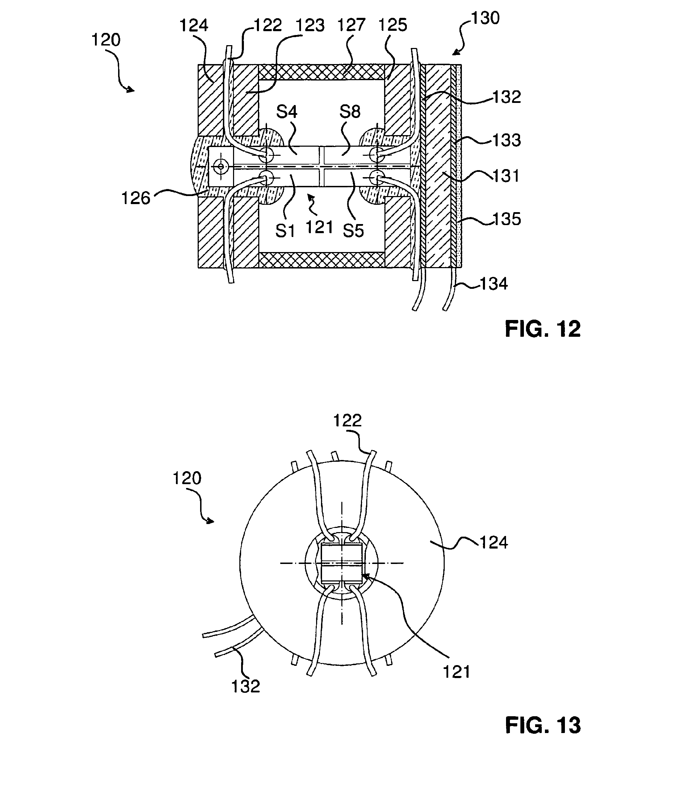

A ninth embodiment is illustrated in Figures 12 and 13. The sensor device 120 comprises a separate bimorph force/moment sensor 121 of similar configuration as the sensor of the first embodiment, and a separate ultrasound transducer 130. First and second concentric disks 123, 124 having central bores are slipped over the force/moment sensor 121 in a proximal end region, the disk axis coinciding with the long axis of the force/moment sensor 121 (the z axis). Between these disks, connecting wires 122 are guided. The space between the disks and the sensor is filled with an adhesive mass 126. A third disk 125 is likewise mounted in a distal end region of the force/moment sensor 121. The disks serve as force induction elements to transfer forces and moments to the bimorph and to facilitate mounting the device on some base. A cylindrical tube 127 made of a relatively soft, pliable and compressible material such as silicone rubber serves as a protective sleeve in order to protect the electrodes of the bimorph. An ultrasound transducer is mounted on disk 125. The transducer comprises a disk-shaped piezoelectric substrate 131 to which a proximal electrode 132 and a distal electrode 133 connected to wires 134 are applied. An acoustic impedance matching layer 135 is disposed on the distal electrode 133. Again, the electric polarization of the force/moment sensor is perpendicular to the electric polarization of the ultrasound transducer, and the ultrasound transducer may be operated in thickness mode. An advantage of this configuration is that a comparatively large ultrasound transducer may be used. The transducer may have different forms, e.g., an annular shape, instead of a disk shape, and/or may be an array transducer.

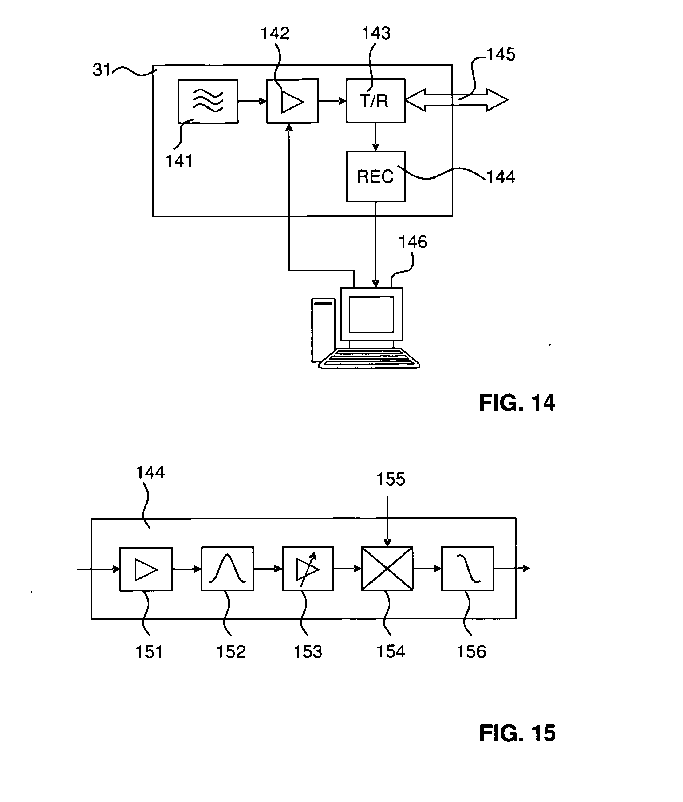

Figures 14 illustrates a possible embodiment of an ultrasound module 31. In this embodiment, the module comprises an oscillator 141 operating in the ultrasound frequency range. The output of the oscillator is fed to a pulse former/amplifier 142 controlled by a computer 146 having a processor. The output of the amplifier 142 is fed to a transmit/receive (T/R) switch 143, which passes the pulses from the amplifier to the sensor device (arrow 145). Echo signals are received by the T/R switch 143 and are passed to a receiver 144. The output of the receiver is then passed to the computer 146 for further processing and displaying. The computer may be adapted, e.g., to display the processed echo signals in any known mode, e.g., in A mode.

The receiver may be set up as shown in Fig. 15. A preamplifier 151 amplifies the weak echo signals. The amplified signals are passed through a bandpass filter 152 centered at the operating frequency of the sensor and to a variable-gain amplifier 153. The output of that amplifier is mixed with the oscillator output 155 from oscillator 141 in a mixer 154 and passed to a low-pass filter 156 to provide a low-frequency output signal.

In summary, the present invention provides sensor devices that are capable of operating as a three-dimensional force sensor and as an ultrasound device. The ultrasound device may be operated in A mode, for measuring parameters like tissue density or thickness in one dimension in the direction of an axis of the device, or in any other known mode known in the art of ultrasound imaging. The functions of force sensing and of ultrasound measurement can be embodied with the same transducer or separated into two different transducers, the one measuring force and the other distance/thickness/tissue density etc.

The sensor device of the present invention may be used in different types of minimally invasive surgeries, for measuring the magnitude and direction of forces that a surgical device applies on the tissue, for the measurement of the spatial angle between the surgical tool and the tissue it contacts and for the measurement of the thickness of the tissue. Because of the reversibility of the piezoelectric effect, the piezoelectric sensor can perform self tests in order to detect malfunctions and determine sensitivity. If operation at a large temperature scale is needed, a thermocouple may be added to the sensor, and the sensor can be calibrated in software. The sensor may be used in a variety of different applications, e.g. as a haptic interface for minimally invasive surgery, as a sensor for feed-back for a medical robot, a sensing element for on the tip of an endoscope etc.

In the following, without wishing to be bound by theory, some exemplary considerations about sensitivity shall be made. A piezoelectric bimorph of the overall dimensions: {*L*,*b*,2*t*} = {4,0.8,08} mm is small enough to be installed at the tip of an instrument for minimally invasive surgery. Using Nolic PCM53 PZT the stress that can be created in the axial direction z is:

σ *_{z}* = *Yd*₃₁*V* = 962.66 Pa,

where: *d*₃₁ = 152 pC/m is the piezoelectric coefficient, *Y* = 63.3 GPa is the Young modulus of the PZT and *V* = 100 V is a typical excitation voltage (the maximal voltage that can be applied on the transducer is 400 V). The voltage can be reduced by using multilayer actuators.

Let us assume that the sensor is used to measure thickness of portion of muscle tissue which is 10 mm thick and bounded by water, and that the attenuation in the muscle is 2 dB/cm/MHz and the acoustic reflection from the muscle-water boundary is 6.33%. Then the pulse reduction will be -64 dB. Producing a 10 MHz pulse will result in a 6.5 mV return pulse in the sensor. The axial resolution of the A-mode ultrasound is 0.15 mm and lateral dispersion is 3.5 mm. This estimation is based on a single point transmitter with reflection from the shortest distance and no additional point. This assumption is conservative because other points in the proximity of the closest point will also have reflections which will increase the basic response signal. These considerations show that the sensors of the present invention may be readily integrated into a surgical instrument.

It will be apparent to a person skilled in the art that many variations of the above-described embodiments are possible and that the invention is not restricted to the above examples. In particular, other electrode configurations are possible, and other detection circuits may be conceived.

### LIST OF REFERENCE SIGNS

- 10: sensor device
- 11: first layer
- 12: second layer
- 13: upper electrode
- 14: lower electrode
- 15: central electrode
- 16: wire
- 17: soldering point

- 18: matching element
- 20: sensor device
- 21: piezoelectric tube
- 22: bore
- 23: outer electrode
- 25: inner electrode
- 26: wire
- 27: soldering point
- 28: matching element
- 30: signal processing device
- 31: load evaluation module
- 32: ultrasound module
- 33: multiplexer
- 40: sensor device
- 41: force/moment sensing section
- 42: ultrasound section
- 43: upper electrode
- 44: lower electrode
- 46: wire
- 50: sensor device
- 51: force/moment sensing section
- 52: ultrasound section
- 53: outer electrode
- 54: inner electrode
- 56: wire
- 60: signal processing device
- 61: load evaluation module
- 62: ultrasound module
- 70: sensor device
- 71: force/moment sensing section
- 72: ultrasound section
- 73: upper electrode
- 74: lower electrode
- 76: wire
- 80: sensor device
- 81: force/moment sensing section
- 82: ultrasound section
- 83: outer electrode
- 86: wire

- 90: signal processing device
- 91: load evaluation module
- 92: ultrasound module
- 100: sensor device
- 101: force/moment sensing section
- 102: ultrasound section
- 104: through-hole
- 105: central electrode
- 110: sensor device
- 111: ultrasound transducer
- 112: piezoelectric body
- 113: first electrode
- 114: second electrode
- 115: matching layer
- 120: sensor device
- 121: force/moment sensor
- 122: wire
- 123: first disk
- 124: second disk
- 125: third disk
- 126: adhesive mass
- 127: protective tube
- 130: ultrasound transducer
- 131: piezoelectric substrate
- 132: first electrode
- 133: second electrode
- 134: wire
- 135: matching element
- S1-S8: electrodes
- S1'-S8': electrodes

## Claims

1. A sensor device (10; 20; 40; 50; 70; 80; 100; 110; 120) comprising:
one or more piezoelectric elements (11, 12; 21; 131) consisting essentially of a piezoelectric material having a first acoustic impedance;
a set of electrodes (13, 14; 23, 24; 43, 44; 53, 54; 73, 74; 83, 84; 113; 114, 132, 133), the electrodes being arranged on said piezoelectric elements in a manner to allow to excite and detect an ultrasonic sound field at least along a first direction and to allow to collect and distinguish charges on said piezoelectric elements resulting from forces along at least a second and a third direction perpendicular to the first direction; and
an acoustic impedance matching element (18; 28; 115; 135) arranged on an ultrasound excitation surface of at least one of said piezoelectric elements for matching an acoustic impedance of said piezoelectric material to an acoustic impedance of a surrounding medium.

2. The sensor device of claim 1, wherein the set of electrodes (13, 14; 23, 24; 43, 44; 53, 54; 73, 74; 83, 84; 113; 114, 132, 133) comprises a first subset of electrodes for exciting and detecting ultrasonic vibrations and a second subset of electrodes for collecting charges resulting from said forces, and wherein at least some of the electrodes of the first and second subset are disposed on a common piezoelectric element.

3. The sensor device of claim 2, wherein the first and second subset have at least some of said electrodes in common.

4. The sensor device of any of the preceding claims, having a generally elongated shape with a long axis defining said first direction, and having a proximal and a distal end relative to the long axis, wherein the acoustic impedance matching element is disposed at the distal end of the sensor device.

5. The sensor device of any of the preceding claims, wherein the electrodes (13, 14; 23, 24; 43, 44; 53, 54; 73, 74; 83, 84; 113; 114, 132, 133) are arranged on said piezoelectric elements in a manner to allow to collect and distinguish charges on said piezoelectric elements resulting from forces along the first direction, in addition to forces along the second and third directions.

6. The sensor device of any of the preceding claims, wherein the electrodes are arranged on said piezoelectric elements in a manner to allow to adjust a direction of said sound field relative to said piezoelectric element.

7. The sensor device of claim 6, wherein the electrodes comprise at least one group of electrodes (73, 74; 83, 84) arranged in a repetitive arrangement along a direction that is substantially orthogonal to the first direction.

8. The sensor device of any of the preceding claims, comprising a piezoelectric bimorph having a first and a second piezoelectric layer (11, 12),
wherein the set of electrodes comprises a central electrode (15; 105) arranged between the first and the second piezoelectric layer, and
wherein the set of electrodes comprises a plurality of electrodes (13, 14) arranged on outer surfaces of said first and second layer facing away from said central electrode, for exciting an ultrasound field and for collecting charges resulting from said forces.

9. The sensor device of claim 8, wherein the bimorph has an elongated shape defining a long axis, a proximal and a distal end, and wherein the set of electrodes comprises:
a first group of at least two electrodes for collecting charges resulting from forces, arranged on the outer surface of the first piezoelectric layer;
a second group of at least two electrodes for collecting charges resulting from forces, arranged on the outer surface of the second piezoelectric layer, each electrode of the second group facing an electrode of substantially identical size and shape of the first group;
a third group of at least two electrodes (43; 44) for exciting the ultrasound field, the third group being arranged on said first and second piezoelectric layers in a distal end region of said bimorph.

10. The sensor device of any of claims 7 to 9, wherein the central electrode (105) is hollow, providing a through-hole along the long axis.

11. The sensor device of any of the preceding claims, wherein the piezoelectric elements comprise a hollow cylindrical tube (21) consisting essentially of a piezoelectric material, the tube having a long axis, and inside peripheral surface and an outside peripheral surface, the set of electrodes comprising at least one inner electrode arranged on said inside peripheral surface and a plurality of electrodes arranged on said outside peripheral surface.

12. The sensor device of claim 11, wherein the set of electrodes comprises:
a group of at least four electrodes for collecting charges resulting from forces, distributed over a circumference of the outer surface of the tube; and
at least one electrode for exciting the ultrasound field, said electrode being arranged on the outer surface of the tube in a distal end region of said tube.

13. A sensor system, comprising a sensor device according to any of the preceding claims and a signal processing device (30; 60; 90) electrically coupled to the electrodes of said sensor device, the signal processing device comprising
an ultrasound module (32; 62; 92) adapted to provide voltages to said electrodes in a manner to excite an ultrasonic vibration of at least one of said piezoelectric elements, to detect charge oscillations on the same piezoelectric element corresponding to at least one ultrasonic return signal in response to said ultrasonic vibration, and to determine an output signal representing said ultrasound return signal; and
a load evaluation module (31; 61; 91) adapted to detect charges collected by the electrodes on at least one of said piezoelectric elements and to determine, from said charges, at least two output signals (F) representing said forces along the second and the third direction.

14. A sensor system comprising:
a sensor device comprising one or more piezoelectric elements (11, 12; 21; 131) and a set of electrodes (13, 14; 23, 24; 43, 44; 53, 54; 73, 74; 83, 84; 132, 133), the electrodes being arranged on said piezoelectric elements in a manner to allow to excite and detect excite an ultrasonic sound field at least along a first direction and to allow to collect and distinguish charges on said piezoelectric elements resulting from forces along at least a second and a third direction perpendicular to the first direction; and
a signal processing device (30; 60; 90) electrically coupled to said electrodes, the signal processing device comprising an ultrasound module (32; 62; 92) adapted to provide voltages to said electrodes in a manner to excite an ultrasonic vibration of at least one of said piezoelectric elements, to detect charge oscillations on the same piezoelectric element corresponding to at least one ultrasonic return signal in response to said ultrasonic vibration, and to determine an output signal representing said ultrasound return signal; and a load evaluation module (31; 61; 91) adapted to detect charges collected by the electrodes on at least one of said piezoelectric elements and to determine, from said charges, at least two output signals (F) representing said forces along the second and the third direction.

15. A method of obtaining information about an object along a first direction and obtaining force information along at least two directions perpendicular to the first direction, the method comprising:
providing a sensor device comprising one or more piezoelectric elements (11, 12; 21; 131) and a set of electrodes (13, 14; 23, 24; 43, 44; 53, 54; 73, 74; 83, 84; 132, 133), the electrodes being arranged on said piezoelectric elements in a manner to allow to excite and detect an ultrasonic sound field at least along a first direction and to allow to collect and distinguish charges on said piezoelectric elements resulting from forces along at least a second and a third direction perpendicular to the first direction;
providing voltages to said electrodes in a manner to excite an ultrasonic vibration of said piezoelectric elements;
detecting charge oscillations on said piezoelectric elements corresponding to at least one ultrasonic return signal in response to said ultrasonic vibration; and
detecting charges collected by the electrodes and determining, from said charges, at least two output values (F) representing said forces along the second and the third direction.
